# EUROPEAN PATENT APPLICATION

(11) **EP 3 685 858 A1**
(43) Date of publication of application: **29.07.2020**
(21) Application number: 19305093.7
(22) Date of filing: 24.01.2019
(51) Int. Cl.: A61K 45/06, A61K 31/138, A61P 11/00

(54) **A NORADRENERGIC SYSTEM STIMULATOR IN THE TREATMENT OF CENTRAL HYPOVENTILATION SYNDROMES**

(71) Applicant: ASSISTANCE PUBLIQUE-HOPITAUX DE PARIS, 75184 Paris Cedex 04 (FR); Université Paris Diderot Paris 7, 75013 Paris (FR)
(72) Inventor: TRANG, Ha, 94410 Saint-Maurice (FR)
(74) Representative: IPAZ

(57) **Abstract**

The present invention concerns a noradrenergic system stimulator and/or a serotonergic system stimulator for its use as medicament in the treatment of central hypoventilation syndromes.

## Description

The present invention concerns a medicament in the treatment of central hypoventilation syndromes.

Central hypoventilation syndromes (CHS) are central autonomic respiratory control and global dysfunction of the autonomic nervous system. Respiration and the respiratory system are under two main control systems: the cortex which provides voluntary or behavioural control, and the bulbo-pontine in the brain stem which provides autonomic or chemical control. In CHS patients, central chemoreceptors are less sensible to the variation of Pco₂, which causes reduced increase of ventilation with regard to increasing of the level of Pco₂. This results in alveolar hypoventilation during spontaneous breathing or ventilatory dependency due to the absence or reduction of a ventilatory response to CO₂.

Central hypoventilation syndromes can either be congenital or acquired later in life. Congenital central hypoventilation syndrome (CCHS), also called as Ondine's curse, is a group of CHS clinically and genetically well-characterized. Based on genetic studies from CCHS patients, it has been identified that the mutations in the *PHOX2B* gene are implied in CCHS (Amiel et al., Nat Genet 2003, 33(4): 459-461; Weese-Mayer et al., AM J Med Genet A 2003, 123A(3):267-278). *PHOX2B* gene is a key player in the prenatal development of the nervous system. Mutations of this gene lead to impaired function of the PHOX2B protein.

Other principal types of central hypoventilation syndromes which can be cited are central hypoventilation with hypothalamic dysfunction, obesity hypoventilation syndrome, idiopathic chronic hypoventilation.

Up to date, no pharmacological treatment is yet available for central hypoventilation syndromes.

Actually, clinically employed treatment relies on respiratory support with either mechanical ventilation via a tracheostomy cannula in severe cases of central hypoventilation syndromes or via a mask, or using phrenic nerve electrical stimulation.

A recent clinical observation has documented a positive response in CCHS patients taking the progestin drug, desogestrel (Straus et al., Respir Physiol Neurobiol 2010, 171(2): 171-174). However, this treatment can only be used in female patients and has not yet shown clinical efficiency.

US 6,331, 536 described a method of treating sleep-related breathing disorders, essentially obstructive or central sleep apneas, by administering an effective amount of a serotonin receptor antagonist or a combination of a serotonin receptor antagonist and a serotonin receptor agonist. This treatment was based on the observation disclosed in this document that administration of serotonin receptor antagonists, caused suppression of central apneas during non-rapid eye movement and especially during rapid eye movement sleep and increased respiratory drive.

However, these results obtained from central apneas cannot be transposed to the treatment of central hypoventilation syndromes, since physiopathological mechanism of central hypoventilation syndromes is fundamentally different from that of central sleep apneas.

In fact, contrary to the case of central hypoventilation syndromes, wherein chemoreceptors have reduced or no response to the increase of Pco₂, in individuals suffering from central sleep apnea, their peripheral and central chemoreceptors show high sensitivity to the change of Pco₂, which puts them in a situation of unstable breathing patterns because they "overrespond" to small changes in chemical stimuli. (Eckert et al., Chest. 2007; 131(2): 595-607)

US 2006/0039866 described a method of treating sleep-related breathing disorders, essentially sleep apnea, by administrating to a patient a therapeutically effective combination of mirtazapine, that is a 5HT3 antagonist, and a second pharmaceutically active ingredient which increase upper airway muscle tone during sleep and stabilizes respiratory drive. The examples of this document only demonstrate the effects of mirtazapine used in monotherapy or in combination with another pharmaceutically active ingredient, such as zonisamide, which has the function to increase upper airway muscle tone during sleep. This effect is useful for treating obstructive apnea since it implies mostly the obstruction of upper airway.

In view of the fact that the only efficiently treatment of central hypoventilation syndromes still relies on respiratory support with mechanical ventilation, it remains a need for developing a simple pharmacologically-based treatment which can be used for treating central hypoventilation syndromes of all kinds of population.

The purpose of the present invention is to provide a new pharmacological treatment for central hypoventilation syndromes.

The present invention is based on incidental observation made by the Inventor that atomoxetine, which is known as a selective norepinephrine (noradrenaline) reuptake inhibitor and is indicated for use in patients with attention-deficit hyperactivity disorder (ADHD), can significantly increase ventilatory response to CO₂ in patients suffering from congenital central hypoventilation syndrome.

Further studies of the Inventors show that the stimulation of noradrenergic system or of serotonergic system or simultaneous stimulations of noradrenergic system and of serotonergic system can increase ventilatory response to CO₂ in patients suffering of central hypoventilation syndromes.

The first subject matter of the present invention concerns the use of a noradrenergic system stimulator and/or a serotonergic system stimulator as a medicament in the treatment of central hypoventilation syndromes.

According to the 3^{rd} edition of International Classification of Sleep Disorders (American Academy of Sleep Medicine), central hypoventilation syndromes comprises following subtypes: (i) congenital central hypoventilation syndrome, (ii) obesity hypoventilation syndrome, (iii) idiopathic central alveolar hypoventilation, (iv) late-onset central hypoventilation with hypothalamic dysfunction, (v) Sleep-related hypoventilation due to a medication or substance, (vi) sleep-related hypoventilation due to a medical disorder.

Central hypoventilation syndromes are characterized by abnormally elevated arterial carbon dioxide partial pressure (Pco₂) during sleep. For adults, a hypoventilation is defined by an increase of Pco₂ level to more than 55mm Hg during at least 10 minutes or an increase of at least 10 mm Hg above the waking value and for a value of at least 50 mm Hg during at least 10 minutes. For children, a hypoventilation is defined by the value of Pco₂ higher than 50 mm Hg during more than 25% of total sleeping time.

The expression "noradrenergic system stimulator" as used herein is meant to a chemical or a biological molecule which has effect of stimulating or activating neurotransmission mediated by noradrenaline (NA).

Noradrenaline is also called norepinephrine (NE). The expressions "noradrenaline" and "norepinephrine" are interchangeable in the present invention.

The effect of a noradrenergic system stimulator is to increase the level of norepinephrine in synaptic space.

Within the meaning of the present invention, a noradrenergic system stimulator can be a chemical or a biological molecule which increases the release of norepinephrine, or inhibits its metabolic degradation, or inhibits its reuptake mediated by norepinephrine transporter.

Alpha-1 adrenergic agonists or alpha-2 adrenergic antagonists are examples of molecules which can increase the release of norepinephrine.

Alpha-1 adrenergic agonists are any molecules that bind to and stimulate the alpha-1 adrenergic receptor.

The function of alpha-1 adrenergic agonist is to increase noradrenergic neurotransmission.

Examples of alpha-1 adrenergic agonist include, but are not limited to dihydroergotamine, etilefrine, ergotamine, midodrine, phenylephrine, methylergometrine.

Alpha-2 adrenergic antagonists are any molecules that bind to alpha-2 adrenergic receptors and therefore block the activation of these receptors by norepinephrine or other ligand.

Alpha-2 adrenergic antagonist is also called alpha-2 blocker.

The function of alpha-2 adrenergic antagonist is to increase noradrenergic neurotransmission.

Examples of alpha-2 adrenergic antagonists include, but are not limited to yohimbine, isoyohimbine, phenoxybenzamine, phentolamine, tolazoline, terazosin, doxazosin, trimazosin, indoramin, ARC239, prazosin, idazoxan, efaroxan, atipamezole.

Examples of molecules which can inhibit metabolic degradation of norepinephrine are inhibitors of mitochondrial monoamine oxidase (IMAO), namely moclobemide, selegiline, iproniazide, toloxatone, rasagiline.

The molecules that inhibit norepinephrine reuptake mediated by norepinephrine transporter are norepinephrine reuptake inhibitors.

Said inhibitor can be a selective norepinephrine reuptake inhibitor, or a norepinephrine reuptake inhibitor with activity at others site, or a serotonin-norepinephrine reuptake inhibitor.

Examples of norepinephrine reuptake inhibitor include, but are not limited to atomoxetine, amedalin, CP-39,332, daledalin, edivoxetine, esreboxetine, lortalamine, nisoxetine, reboxetine, talopram, talsupram, tandamine, viloxazine, brupropion, ciclazindol, manifaxine, maprotiline, radafaxine, tapentadol, teniloxazine, tricyclic andidepressants, desvenlafaxine, duloxetine, levomilnacipran, milacipran, sbrutramine, tramodal, venlafaxine, mazindol.

The expression "serotonergic system stimulator" as used herein is meant to a chemical or a biological molecule which has effect of stimulating or activating neurotransmission mediated by serotonin.

The effect of a serotonergic system stimulator is to increase the level of serotonin in synaptic space.

Serotonin is also called 5-hydroxytryptamine.

Within the meaning of the present invention, a serotonergic system stimulator can be a chemical or a biological molecule which increases the release of serotonin, or inhibits its metabolic degradation, or inhibits its reuptake mediated by serotonin transporter, or serotonergic agonists, or a serotonin reuptake inhibitor.

Serotonergic agonists are examples of molecules which can increase the release of serotonin.

Within the frame of the present invention, serotonergic agonists are chosen from agonist of serotonin 1A receptor, agonist of serotonin 3 receptor, or agonist of serotonin 5 receptor.

Serotonin 1A receptor is also named 5-HT1A receptor. Serotonin 3 receptor is also called 5-HT 3 receptor. Serotonin 5 receptor is also called 5-HT 5 receptor.

Examples of agonist of serotonin 1A receptor that can be cited are buspirone, eptapirone, lesopitron, LY-293,284, repinotan, flibanserin, alnespirone, befiradol, F-15, 599, osemozotan, U-92, 016-A, vortioxetine.

Examples of agonist of serotonin 3 receptor are cereulide, 2-methyl-5-HT, alpha-methyltryptamine, bufotenin, chlorophenylbiguanide, ibogaine, phenylbiguanide, quipazine, RS-56812, SR-57227, varenicline, YP-31636.

Examples of agonist of serotonin 5-receptor are flibanserin, almotriptan, naratriptan, eletriptan, flibanserin, frovatriptan, sumatriptan, rizatriptan, zolmitriptan.

Examples of molecules which can inhibit metabolic degradation of serotonin are inhibitors of mitochondrial monoamine oxidase (IMAO), namely moclobemide, selegiline, iproniazide, toloxatone, rasagiline.

Molecules that inhibit serotonin reuptake mediated by serotonin transporters are serotonin reuptake inhibitors.

Said serotonin reuptake inhibitor can be a selective serotonin reuptake inhibitor, or a serotonin reuptake inhibitor with activity at others site, or a serotonin-norepinephrine reuptake inhibitor.

Examples of serotonin reuptake inhibitor include, but are not limited to citalopram, dapoxetine, escitalopram, paroxetine, desmethylcitalopram, femoxetine, fluoxetine, fluvoxamine, norfluoxetine, paroxetine, sertraline, desmethylsertraline, zimelidine, levomilnacipran, venlafaxine, vilazodone, vortioxetine, amitriptyline, clomipramine, imipramine, mazindol.

According to an embodiment, the present invention concerns a noradrenergic system stimulator as defined above for its use as a medicament in the treatment of central hypoventilation syndromes.

In order to obtain optimistically therapeutic effect, the noradrenergic system stimulator used in the present invention can be a single type or a combination of several types of noradrenergic system stimulators as described above.

In a particular embodiment, the noradrenergic system stimulator is a norepinephrine reuptake inhibitor or an alpha-1 adrenergic agonist, or a combination thereof.

In a more particular embodiment, said norepinephrine reuptake inhibitor is a selective norepinephrine reuptake inhibitor.

A preferred selective norepinephrine reuptake inhibitor for the present invention is atomoxetine.

A preferred embodiment of the present invention concerns a selective norepinephrine reuptake inhibitor, more particularly atomoxetine, for its use as medicament in the treatment of central hypoventilation syndromes.

In another particular embodiment, said norepinephrine reuptake inhibitor is a serotonin-norepinephrine reuptake inhibitor.

In preferred embodiment, the present invention concerns mazindol for its use as medicament in the treatment of central hypoventilation syndromes.

According to another embodiment, the present invention concerns a serotonergic system stimulator as defined above for its use as a medicament in the treatment of central hypoventilation syndromes.

The serotonergic system stimulator used in the present invention can be a single type or a combination of several types of serotonergic stimulator as described above.

In a particular embodiment, the invention concerns the serotonergic system stimulator for its use as medicament in the treatment of central hypoventilation syndromes, wherein the serotonergic system stimulator is a serotonin reuptake inhibitor or a serotonergic agonist, or a combination thereof.

According to still another embodiment, the present invention concerns a noradrenergic system stimulator and a serotonergic system as defined above for their use as a medicament in the treatment of central hypoventilation syndromes.

In a particular embodiment, the invention concerns a combination of a selective norepinephrine reuptake inhibitor with a selective serotonin reuptake inhibitor or with an alpha-1 adrenergic agonist for its use as medicament in the treatment of central hypoventilation syndrome.

In a more particular embodiment, the invention concerns a combination of atomoxetine with a selective serotonin reuptake inhibitor or with an alpha-1 adrenergic agonist for its use as medicament in the treatment of central hypoventilation syndrome.

Said selective serotonin reuptake inhibitor may be chosen from fluoxetine, paroxetine, sertraline, citalopram, escitalopram, indalpine, zimelidine, dapoxetine, fluvoxamine, mirtazapine; said alpha-1 adrenergic agonist is chosen from dihydroergotamine, etilefrine, ergotamine, midodrine, phenylephrine, methylergometrine.

Another aspect of the present invention is to provide a pharmaceutical composition comprising a therapeutically effective amount of a noradrenergic system stimulator and/or a serotonergic system stimulator, for its use in the treatment of central hypoventilation syndromes.

In a particular embodiment of the invention, the pharmaceutical composition comprises a therapeutically effective amount of at least one norepinephrine reuptake inhibitor or a combination of a therapeutically effective amount of a selective norepinephrine reuptake inhibitor with a therapeutically effective amount of a selective serotonin reuptake inhibitor or with a therapeutically effective amount of an alpha-1 adrenergic agonist.

In a more particular embodiment of the invention, the pharmaceutical composition comprises a therapeutically effective amount of atomoxetine or a combination of a therapeutically effective amount of atomoxetine with a therapeutically effective amount of a selective serotonin reuptake inhibitor or with a therapeutically effective amount of an alpha-1 adrenergic agonist.

By the term "therapeutically effective amount" of an above-mentioned compound is referred to an amount of that compound which is tested or calculated as physiologically acceptable for human beings and produces desirable therapeutic effect.

Pharmacology studies of most of norepinephrine reuptake inhibitors, serotonin reuptake inhibitors, alpha-1 adrenergic agonists, alpha-2 antagonists, or serotonergic agonists mentioned above are available in the prior art. A skilled person in the art can determine without undue difficulty the needed effective amount of said compound according to patient's age, weight, and pathology severity.

According to the present invention, the effective amount of a selective norepinephrine reuptake inhibitor is from 10 -100 mg/day; the effective amount of an alpha-1 adrenergic agonist is from 10-100 mg/day, the effective amount of a selective serotonin reuptake inhibitor is from 10-100 mg/day; the effective amount of a combination of a selective norepinephrine reuptake inhibitor and a selective serotonin reuptake inhibitor is from 10 -100 mg/day of a selective norepinephrine reuptake inhibitor and from 10-100 mg/day of a selective serotonin reuptake inhibitor; the effective amount of a combination of a selective norepinephrine reuptake inhibitor and an alpha-1 adrenergic agonist is from 10 - 100 mg/day of a selective norepinephrine reuptake inhibitor and from 10-100 mg/day of an alpha-1 adrenergic agonist.

In a particular embodiment of the present invention, the effective amount of an atomoxetine is from 10-100 mg/day.

The pharmaceutical composition of the present invention can also comprise any suitable pharmaceutically acceptable vehicle.

The choice of suitable pharmaceutically acceptable vehicle depends on the galenic formulation and the administration route of the pharmaceutical composition.

In a preferred embodiment, the pharmaceutical composition of the present invention is formulated in solid form for an oral administration.

In a particular embodiment, the pharmaceutical composition of the present invention is in the form of tablets or capsules.

The pharmaceutical composition of the present invention is used as medicament in the treatment of central hypoventilation syndrome chosen from congenital central hypoventilation syndrome, central hypoventilation with hypothalamic dysfunction, obesity hypoventilation syndrome, idiopathic chronic hypoventilation.

The present invention is illustrated in more detail by the following example.

### Example

Atomoxetine is administrated to a 14 years old girl of who suffers from congenital central hypoventilation and attention deficit hyperactivity disorder.

This girl bears a mutation on *PHOX2B* gene and the ventilator response to CO₂ during waking is decreased to 2.41 ml/min/mmHg/kg of weight (expected value is more than 20).

After a defeat of treatment by Ritaline for ADHD, she is placed under the treatment of atomoxetine for ADHD at age of 14 years old.

It is observed in this girl a very important increase of ventilatory response to CO₂ under the treatment of atomoxetine, since the value of ventilatory response to CO₂ during waking is increased to 187 ml/min/mmHg/kg of weight.

Since atomoxetine is a selective norepinephrine reuptake inhibitor, this result suggests that the stimulation of noradrenergic system could increase ventilatory response to CO₂

## Claims

1. A noradrenergic system stimulator and/or a serotonergic system stimulator for its use as medicament in the treatment of central hypoventilation syndromes.

2. The noradrenergic system stimulator and/or the serotonergic system stimulator for its use according to claim 1, wherein the noradrenergic system stimulator is a norepinephrine reuptake inhibitor or an alpha-1 adrenergic agonist, or a combination thereof; wherein the serotonergic system stimulator is a serotonin reuptake inhibitor or a serotonergic agonist, or a combination thereof.

3. The noradrenergic system stimulator and/or the serotonergic system stimulator for its use according to claim 1 or 2, wherein said norepinephrine reuptake inhibitor is a selective norepinephrine reuptake inhibitor.

4. The noradrenergic system stimulator and/or the serotonergic system stimulator for its use according to claim 1 or 2, wherein said norepinephrine reuptake inhibitor is a serotonin-norepinephrine reuptake inhibitor

5. The noradrenergic system stimulator and/or the serotonergic system stimulator for its use according to claim 1 to 4, wherein said norepinephrine reuptake inhibitor is chosen from atomoxetine, amedalin, CP-39,332, daledalin, edivoxetine, esreboxetine, lortalamine, nisoxetine, reboxetine, talopram, talsupram, tandamine, viloxazine, brupropion, ciclazindol, manifaxine, maprotiline, radafaxine, tapentadol, teniloxazine, tricyclic andidepressants, desvenlafaxine, duloxetine, levomilnacipran, milacipran, sbrutramine, tramodal, venlafaxine, mazindol.

6. The noradrenergic system stimulator and/or the serotonergic system stimulator for its use according to claim 1 to 4, wherein said norepinephrine reuptake inhibitor is atomoxetine.

7. A combination of a selective norepinephrine reuptake inhibitor with a selective serotonin reuptake inhibitor or with an alpha-1 adrenergic agonist for its use as medicament in the treatment of central hypoventilation syndrome.

8. The combination for its use according to claim 7, wherein said selective serotonin reuptake inhibitor is chosen from fluoxetine, paroxetine, sertraline, citalopram, escitalopram, indalpine, zimelidine, dapoxetine, fluvoxamine, mirtazapine; wherein said alpha-1 adrenergic agonist is chosen from dihydroergotamine, etilefrine, ergotamine, midodrine, phenylephrine, methylergometrine.

9. A pharmaceutical composition comprising a therapeutically effective amount of a noradrenergic system stimulator and/or a serotonergic system stimulator, for its use in the treatment of central hypoventilation syndrome.

10. The pharmaceutical composition for its use according to claim 9 comprising a therapeutically effective amount of at least one norepinephrine reuptake inhibitor or a combination of a therapeutically effective amount of a selective norepinephrine reuptake inhibitor with a therapeutically effective amount of a selective serotonin reuptake inhibitor or with a therapeutically effective amount of an alpha-1 adrenergic agonist.

11. The pharmaceutical composition for its use according to claim 10, wherein the effective amount is from 10 -100 mg/day of a norepinephrine reuptake inhibitor, or from 10-100 mg/day of a selective serotonin reuptake inhibitor, or from 10-100 mg/day of an alpha-1 adrenergic agonist, or a combination of from 10 -100 mg/day of a selective norepinephrine reuptake inhibitor and from 10-100 mg/day of a selective serotonin reuptake inhibitor, or a combination of from 10 -100 mg/day of a selective norepinephrine reuptake inhibitor and from 10-100 mg/day of an alpha-1 adrenergic agonist.

12. The pharmaceutical composition for its use according to 11, wherein the central hypoventilation syndrome is chosen from congenital central hypoventilation syndrome, central hypoventilation with hypothalamic dysfunction, obesity hypoventilation syndrome, idiopathic chronic hypoventilation.
